# EUROPEAN PATENT SPECIFICATION

(11) **EP 3 897 787 B1**
(45) Date of publication and mention of the grant of the patent: **11.01.2023**
(21) Application number: 19836582.7
(22) Date of filing: 20.12.2019
(51) Int. Cl.: A61M 5/315, A61M 5/32, A61M 5/24

(54) **A PISTON FOR A TUBULAR VIAL, TUBULAR VIAL AND CORRESPONDING INJECTION KIT**
KOLBEN FÜR EINE RÖHRENFÖRMIGE PHIOLE, RÖHRENFÖRMIGE PHIOLE UND ZUGEHÖRIGES INJEKTIONSKIT
PISTON POUR FLACON TUBULAIRE, FLACON TUBULAIRE ET KIT D'INJECTION CORRESPONDANT

(30) Priority: 21.12.2018 IT 201800020905
(43) Date of publication of application: 27.10.2021
(73) Proprietor: Mariani, Umberto, 24042 Capriate San Gervasio (BG) (IT)
(72) Inventor: Mariani, Umberto, 24042 Capriate San Gervasio (BG) (IT)
(74) Representative: Gatti, Enrico
(86) International application number: PCT/IB2019/061183
(87) International publication number: WO 2020/128998

(56) References cited:
- WO-A1-2009/003234
- US-A- 5 401 246

## Description

### Field of the invention

The present invention relates to the medico-surgical sector, and in particular regards re-usable injection kits, such as autoclave sterilizable syringes for dental surgery.

### Prior art and general technical problem

In the medical and surgical field, recourse is frequently had to injection kits comprising a multishot syringe loaded with a tubular vial (which constitutes the cartridge thereof) containing a drug to be administered to a patient. Administration is made by movement of a piston that is able to slide in a fluid-tight way inside the tubular vial. In the dentistry sector, such injection kits find widespread use in administration of anaesthetics.

Operatively, administration of the drug takes place by coupling a disposable sterile needle to the body of the syringe. The syringe generally comprises a tubular cradle, in which the tubular vial is received at the moment of loading, and a slider, which is axially mobile within the cradle. The tubular vial comprises a tubular casing, typically made of glass, the ends of which are closed, one by a perforable diaphragm and the other by a piston that is able to slide axially within the casing. The piston is engaged by the slider to cause an axial movement thereof towards the end where the diaphragm is present, while the diaphragm is perforated by the sterile needle at the moment of coupling thereof to the cradle of the syringe (generally provided by way of an ogee shaped element) .

The most critical step of use of such an injection kit comprises the instants that follow injection, and in particular when the needle contaminated with the patient's blood is set in a condition where it does not constitute a hazard (de-risking).

Currently, the majority of medical staff simply carries out manual capping of the needle by re-using the same protective cap that constitutes the sterile casing of the needle, exposing themselves, however, to an extremely high risk of injury.

It is sufficient, in fact, to fail to align properly the cap and the needle to suffer injury to the palm or fingers of the hand, with potentially extremely serious consequences in the case where the blood were a carrier of infectious illnesses.

The inventor has already proposed a solution to the above technical problem in the Italian patent application for industrial invention No. 102016000010073. This solution envisages a modification of the piston of the tubular vial by providing an axial extension that is able to couple by snap action with the sterile needle, which is also modified accordingly.

In the solution in question, at the end of the injection stroke, the piston of the tubular vial couples to the needle via engagement by snap action between the axial extension and the end of the needle that has perforated the diaphragm of the tubular vial. The piston is then retracted via reversal of the motion of the slider, thus drawing the needle within the tubular vial and encasing it in a safe and secure way.

In normal practice, the above solution has highlighted, however, a non-negligible problem: in the proximity of the end of the injection stroke there remains a certain amount of liquid drug trapped between the piston and the diaphragm, in particular trapped in a portion no longer exposed to the mouth of the needle inside the tubular vial. In certain circumstances, this renders impossible coupling by snap action between the axial extension of the piston and the end of the sterile needle that faces the inside of tubular vial as a result of the incompressibility of the liquid. For this reason, in the patent application in question, the inventor has proposed, as further solution, provision of a relief or by-pass channel extending in a direction transverse to the longitudinal axis of the piston and passing through it, where the channel is provided along the axial extension.

Notwithstanding this, a solution of this type is not yet optimal. The technological modalities of production of the piston in question are such that the through channel requires provision of die slides. This is required because the transverse orientation of the relief channel (imposed by functional needs) creates an undercut that prevents removal of the moulded item from the mould in the absence of die slides.

Unfortunately, considering the tiny dimensions of the component in question, the use of die slides in the mould frequently gives rise to moulding burrs, which may create partial or total occlusions of the relief channel, neutralising the effectiveness thereof.

### Object of the invention

The object of the present invention is to solve the technical problems mentioned previously and is defined by the subject-matter of independent claim 1. Advantageous embodiments of the invention are described in the dependent claims.

In particular, the object of the invention is to provide a piston, a tubular vial, and a corresponding injection kit that will eliminate the risks of injury for the operator and that will likewise be immune from malfunctioning.

### Summary of the invention

The object of the present invention is achieved by a piston, a tubular vial, and an injection kit that present the characteristics that form the subject of the ensuing claims, which form an integral part of the technical disclosure provided herein in relation to the invention.

### Brief description of the drawings

The invention will now be described with reference to the annexed drawings, which are provided purely by way of non-limiting example and in which:
- Figure 1 is a perspective view of a piston according to the invention;
- Figure 2 is a view according to the arrow II of Figure 1;
- Figure 3 is a cross-sectional view according to the trace III-III of Figure 2;
- Figure 4 is a cross-sectional view according to the trace IV-IV of Figure 2;
- Figure 5 is a longitudinal sectional view of a tubular vial according to the invention;
- Figures 6, 7, 8, and 9 each represent a longitudinal sectional view of an injection kit according to the invention, where each figure illustrates a corresponding step of an operating sequence;
- Figures 6A and 7A are enlarged views of the conditions illustrated in Figures 6 and 7; and
- Figure 10 and Figure 11 are enlarged longitudinal sections of the injection kit according to the invention and illustrate, respectively, a condition immediately preceding and a condition coinciding with the condition of Figure 7, whereas Figure 10A is an enlarged view according to the arrow X of Figure 10.

### Detailed description

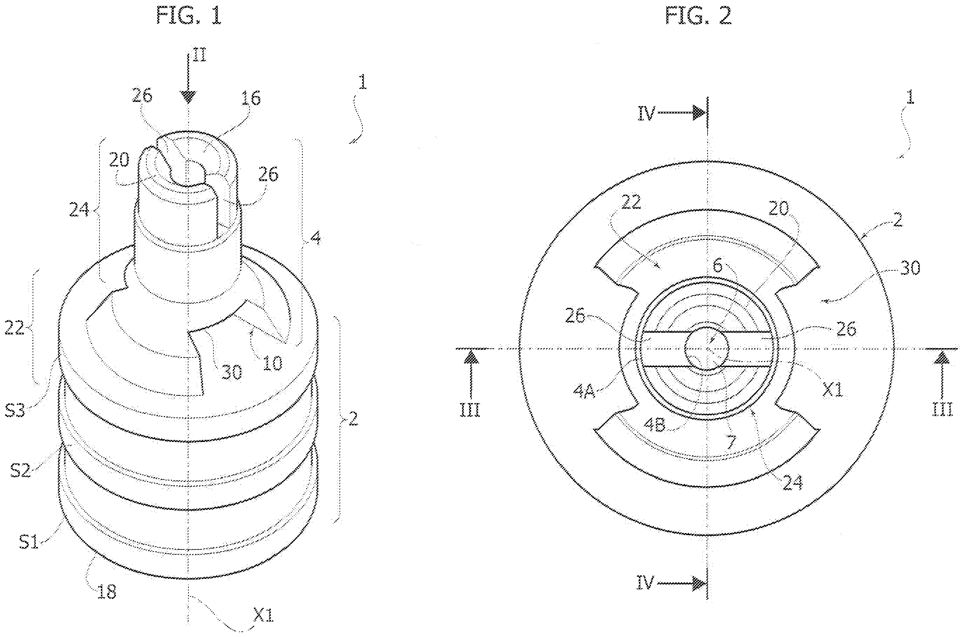

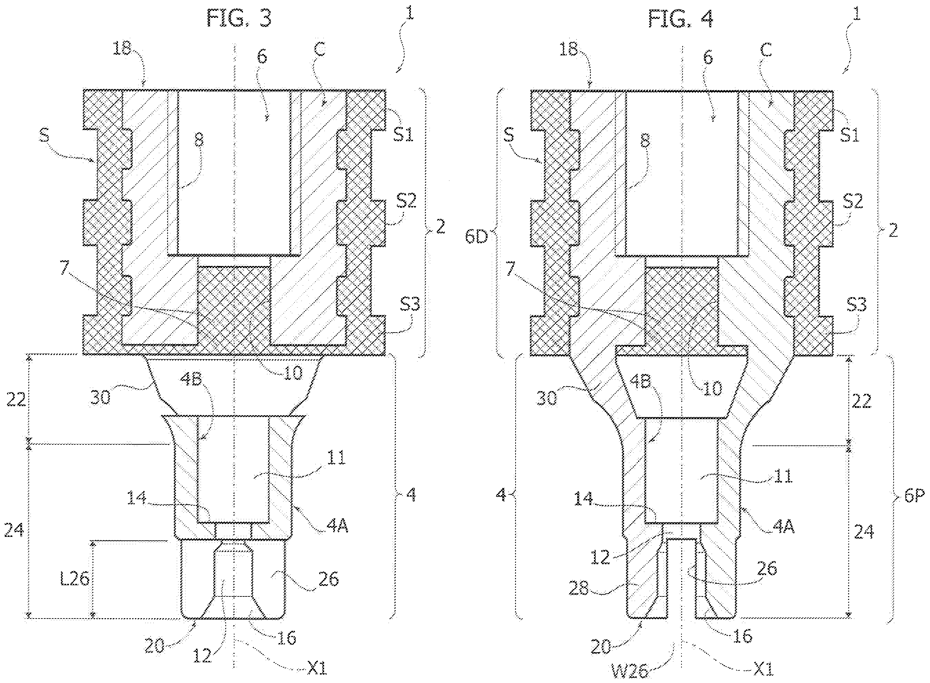

The reference number 1 in Figures 1 to 4 designates as a whole a piston for a tubular vial according to the invention. The piston 1 comprises a skirt 2 and an axial extension 4, adjacent to the skirt 2 and projecting axially beyond the skirt 2. With reference to the sectional views of Figures 3 and 4, preferentially the piston 1 of this sort is provided by co-moulding of two distinct portions. The skirt 2 is configured for guiding the piston along an inner surface of a tubular vial and is moreover configured for providing tightness with respect thereto.

In particular, the piston 1 is provided by co-moulding of a core portion C made of rigid plastic material and a coating portion S made of deformable and/or elastomeric plastic material, where the core portion C provides a core of the skirt 2 and all the axial extension 4, whereas the coating portion S defines the outer layer of the skirt 2 and in particular the layer that guides the piston 1 along the wall of the casing of a tubular vial, and that provides fluid tightness with respect to the wall. For this purpose, the coating portion S comprises one or more circumferential projections S1, S2, S3, which have the function of sealing segments with respect to the inner wall of the casing of a tubular vial.

The piston 1 has a longitudinal axis X1 and is traversed by a longitudinal cavity 6 that extends along the axis X1 throughout the axial length of the piston 1. The cavity 6 is visible in the sectional views of Figures 3 and 4.

The cavity 6 hence extends both within the skirt 2 and within the axial extension 4, but at the interface between them it is rendered blind by a diaphragm 7 made from the same material of the coating portion S at the moment of co-moulding on the core portion C. In this way, once the piston 1 is inserted in a tubular vial, the environments upstream and downstream of the skirt 2 remain isolated, enabling the piston 1 to bring about a displacement of volume of fluid.

With the partition introduced by the diaphragm 7, there may hence be identified two distinct portions of the cavity 6, in particular a first portion 6D (or distal portion, as will emerge more clearly from the ensuing description), and a second portion 6P (or proximal portion, as will emerge more clearly from the ensuing description).

In particular, the portion 6D in turn comprises a first stretch 8 and a second stretch 10, which has a smaller diameter than the first stretch 8. The first stretch 8 preferably comprises a coupling seat provided as threaded hole.

The portion 6P in turn comprises a third stretch 11 and a fourth stretch 12, which has a smaller diameter than the third stretch 11, thus defining a shoulder 14 between them. The fourth stretch 12 moreover preferably terminates with a flaring 16.

Preferentially, the stretch 11 and the stretch 10 are provided as continuous geometry (having the same diameter) on the core element C, the continuity of which is subsequently interrupted at the moment of co-moulding of the coating portion S, with creation of the diaphragm 7.

The cavity 6 (and all the portions thereof) hence exhibits a diameter that decreases progressively from a first axial end 18, located in a point corresponding to the skirt 2, to a second axial end 20, which corresponds to and is located at a free (axial) end of the axial extension 4.

On the basis of the above identification of the portions 6S and 6P, the distribution of the various stretches with respect to the ends 18, 20 is hence the following:
- the stretch 8 extends from the first axial end 18 to a first end of the stretch 10;
- the stretch 10 extends from its own first end as far as a second end defined by the diaphragm 7;
- the diaphragm 7 extends between the second end of the stretch 10 as far as a first end of the stretch 11;
- the stretch 11 extends from its own first end as far as a second end, coinciding with a first end of the stretch 12; and
- the stretch 12 extends from its own first end as far as the axial end 20.

Provision of the cavity 6 hence enables identification of an inner surface and an outer surface for each portion of the piston 1, and in particular the presence of the portion 6P (which is to all effects an axial/longitudinal cavity provided in the axial extension 4) defines for the axial extension 4 an outer surface designated in the figures by the reference 4A, and an inner surface designated by the reference 4B, where the inner surface 4B gives out into the cavity 6P.

Once again on the axial extension 4, there can be identified different portions, in particular a tapered portion 22 and a cylindrical portion 24. The tapered portion 22 extends from the skirt 2 to the cylindrical portion 24, whereas the cylindrical portion 24 extends from the tapered portion 22 to the end 20 of the piston 1. As may be seen in Figure 4, the portion 6P of the cavity 6 preferably extends along the entire axial extension 4.

According to the invention, at the axial end 20 at least one through opening 26 is provided, and preferably two as illustrated in the figures, which extend from the outer surface 4A to the inner surface 4B of the axial extension 4. The intersection of each of the through openings 26 with the outer surface 4A defines an open perimeter, and likewise the intersection of each of the through openings 26 with the inner surface 4B defines an open perimeter. The through openings 26 have an axial length L26 (measured along the axis X1, Figure 3) preferably comprised in the range between 2 mm and 3 mm, and a transverse width W26 (measured in the chordal direction, Figure 4) comprised in the range between 0.3 mm and 0.7 mm. In both ranges the endpoints are understood to be included. Preferred values within the ranges in question are 0.5 mm for the width W26 and 2.3 mm for the length L26.

The openings 26 are, for this purpose, provided in an axial end position on the body of the piston 1 and in particular on the axial extension 4 in a position such that the perimeter of the intersection with the surfaces 4A, 4B is open at the axial end 20.

The openings 26 substantially provide two grooves or notches that extend axially at the axial end 20 of the axial extension 4 (in addition, obviously, to extending through the thickness thereof with a development of a channel type, in particular an exposed channel), and globally bestow thereon a substantially forked shape, with two separate portions having a cylindrical geometry 28 that give out onto the through axial cavity 6. In the case of a single opening 26 a single portion with cylindrical geometry 28 will obviously be created, open in the area of the opening itself, and in general n openings 26 will give rise to n portions 28.

At the tapered portion 22, at least one further through opening 30 is moreover provided, which extends in a direction transverse to the axis X1 and traverses the axial extension 4 from one side to the other of the outer surface 4A. The through opening 30 is preferably provided in a position adjacent to the skirt, and moreover intersects the portion 6P of the cavity 6 sharing the axis X1, giving rise to a substantially T-shaped section of intersection and likewise establishing a fluid communication with the portion 6P.

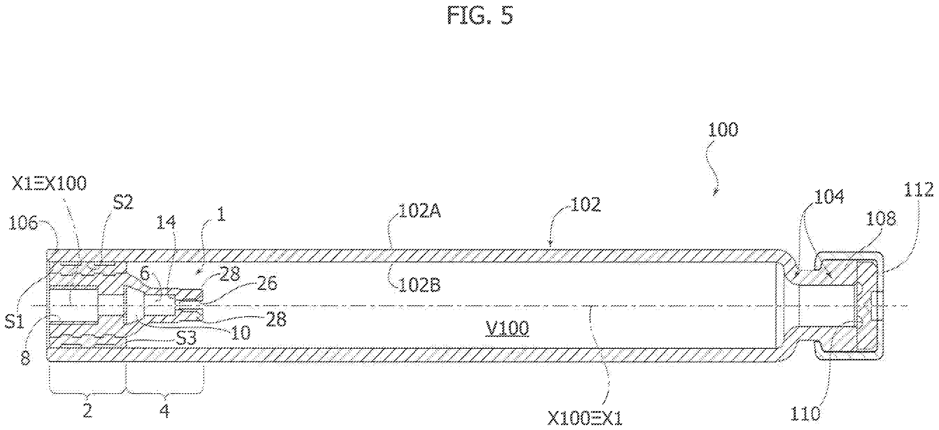

With reference to Figure 5, the reference number 100 designates as a whole a tubular vial according to the invention. The tubular vial 100 comprises a tubular casing 102 having a cylindrical shape with an outer surface 102A, an inner surface 102B, and a longitudinal axis X100, and moreover having a first end 104 and a second end 106. The first end 104 is open and presents a necking so as to define a collar 108, whereas the end 106 is open without any variations of cross section.

Inserted at the end 106 is the piston 1 (the axis X1 comes to coincide with the axis X100), which in the resting condition occupies the position illustrated in Figure 5, where the volume V100 that it faces is maximum. The piston is able to slide axially within the casing 102 along the axis X100 and is moreover configured for providing fluid tightness against the surface 102B thanks to the deformability of the material of the coating portion S, and in particular thanks to the projections S1, S2, S3.

Installed, instead, at the end 104 is a pierceable diaphragm 110 that occludes the end 104 itself, delimiting the volume V100 at the end opposite to the piston 1. The volume V100 is filled with liquid, typically a pharmacological solution (e.g., an anaesthetic).

The diaphragm 110 is withheld on the end 104, in particular on the collar 108, by means of a cap 112 secured to the collar 108 by plastic deformation, and in particular bent inwards so as to grip the collar 108.

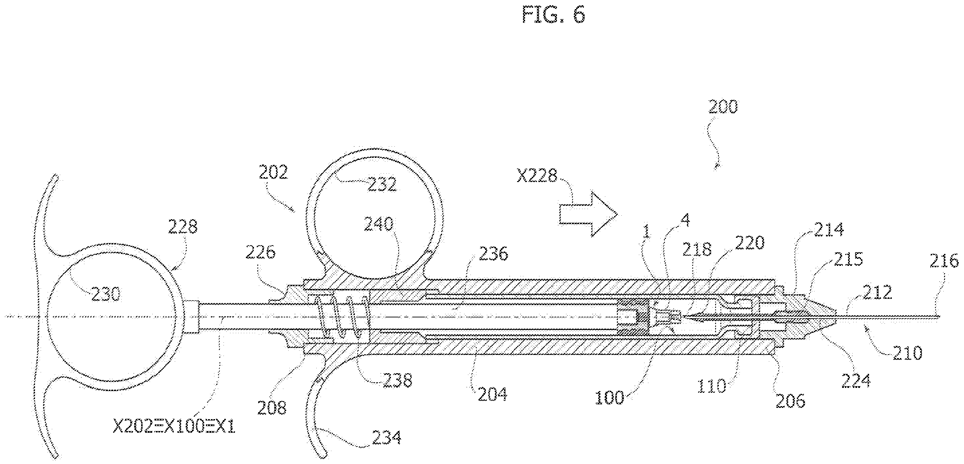

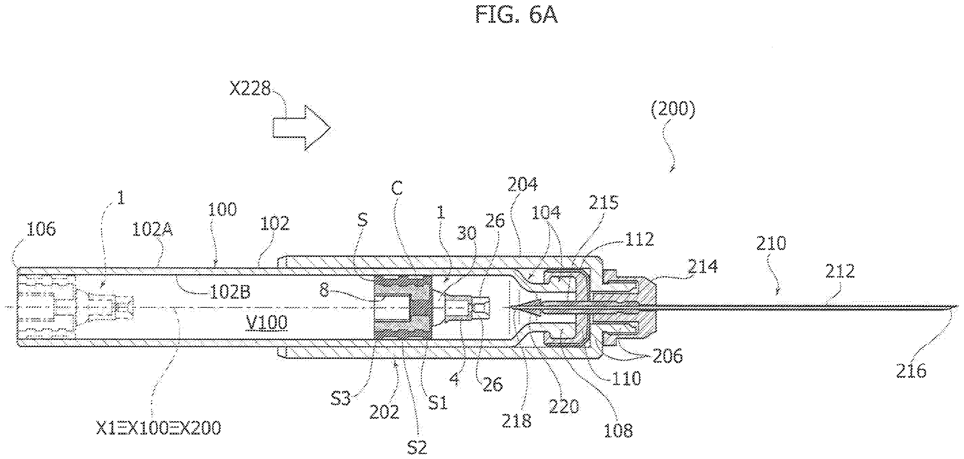

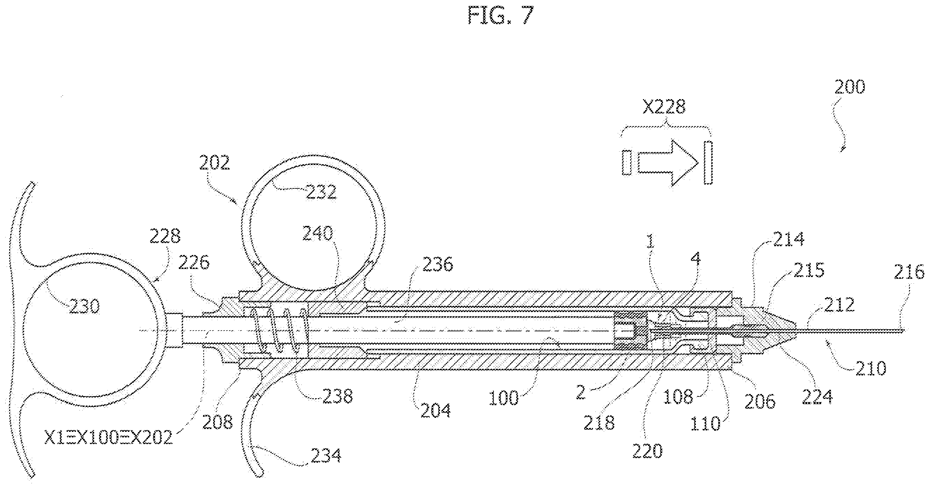

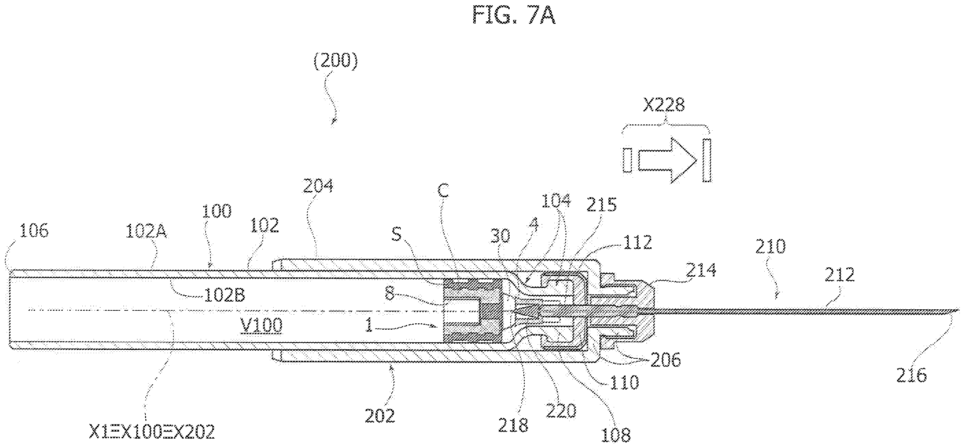

With reference to Figure 6 and to the subsequent Figures 7 to 9, the reference number 200 designates as a whole an injection kit according to the invention. The injection kit 200 comprises an (autoclave sterilizable) re-usable syringe 202 and a tubular vial 100. Consequently, the kit 200 includes the piston 1 according to the invention.

The syringe 202 has a longitudinal axis X202 and comprises a cradle 204 having a cylindrical shape, configured for housing the tubular vial 100, which functions as cartridge for the syringe itself. The cradle 204 develops about the axis X202.

The cradle 204 comprises a proximal end 206 and a distal end 208, where the terms "proximal" and "distal" are understood with reference to the site where the syringe is used. In general, in the context of this description, the terms "proximal" and "distal" are understood with reference to the injection site, the term "proximal" meaning the position facing and/or close to the injection site, and the term "distal" meaning the position facing in the direction opposite to the injection site and/or further away therefrom. Both ends of the cradle 204 are open and function as coupling seat for further components that will be described hereinafter.

The proximal end 206 is configured for receiving a proximal unit 210 comprising a sterile needle 212 and a bushing member 214 (typically having an ogee-like shape), within which the needle 212 is inserted. The proximal end 206 can be provided either with a shape that is simply open, as is represented in Figures 6, 7, 8, and 9, or with a shoulder, necking, and threaded collar, as may be seen in Figure 6A and 7A (in this regard, Figures 6A and 7A are consistent with Figures 6 and 7, from the standpoint of representation of the operating sequence, but present differences in terms of geometry so as to provide a representation of the entire gamut of alternatives, avoiding a massive multiplication of the figures).

In the case of a simply open proximal end 206, this is provided with an internal thread in which the bushing member 241 (which is consequently provided with a corresponding external thread) engages, whereas, in the case of the proximal end 206 with shoulder, necking, and threaded collar, it is the latter that has an external thread on which an internal thread of the bushing member 214 engages.

In greater detail, the needle 212 is inserted in a jacket 215 and comprises a proximal end 216 and a distal end 218. The proximal end 216 extends axially beyond the bushing member 214 and constitutes the end of the needle 212 that penetrates into the into the injection site, whereas the distal end 218 projects distally beyond the jacket 215, and in particular projects distally beyond a distal stud 220 of the jacket 215. The distal stud 220 presents a region with a widened diameter so as to create a shoulder configured for co-operating with the shoulder 12 of the axial extension 4 according to modalities that will be described shortly. Preferentially, the stud 220 has a conical shape with distal tapering so as to divaricate the diaphragm 110 simultaneously with perforation thereof by the distal end 218, thus facilitating penetration of the jacket 215.

The jacket 215 and the needle 212 are rendered fixed with respect to one another, for example, by co-moulding of the latter on the former or else by forming the jacket 215 integrally with the needle 212 (in this case, the ensemble is made entirely of the metal material of the needle), and are inserted as monolithic unit within a through axial cavity 224 of the bushing member 214.

Coupling between the through axial cavity 224 and the jacket 215 is provided in such a way that they are fixed (rigid connection) until a given loading threshold is reached. The loading threshold is chosen in such a way as to maintain the stability of the position of the needle 212 with respect to the bushing member 214 at the moment of injection of the liquid contained in the tubular vial 100 into the patient, and at the same time in such a way as to enable movement in a proximal-to-distal direction of the jacket 215 after the end of injection. This operating mode will be rendered more evident in the ensuing description.

The bushing member 214 can be coupled to the proximal end 206, preferably via a threaded connection (the threads are provided directly on the end 206 and on the bushing member 214). Both the bushing member 214 and the cavity 224 (and as a result the needle 212 and the jacket 215) share the axis X202.

At the distal end 208, the cradle 204 is closed by a guide bearing 226, which functions as guide element for a slider 228 that is slidable in a longitudinal direction along the axis X202. In way in itself known, the slider 228 comprises a first ring 230 in which the thumb of an operator can be inserted and which is configured for co-operating with a second ring 232 provided directly on the body of the cradle 204. The ring 232 is in general designed for insertion of the index finger of the operator, while a bar 234 - which is also provided on the cradle 204 in a diametrally opposite position with respect to the ring 232 - is configured as resting portion for the middle finger of the operator.

The slider 228 further comprises a thrust rod 236, which is configured for engaging the piston 1 and in particular is configured for engaging the portion 8 by means of a male threaded element. The coupling may be provided also in further ways, provided that these allow transmission of bi-directional forces between the piston 1 and the rod 236 (distal-to-proximal and proximal-to-distal forces).

An elastic element 238 is fitted on the thrust rod 236 and is withheld between the guide bearing 226 and a sliding bearing 240, the latter functioning as breech for loading of the tubular vial 100. In particular, the bearing 240 is configured for being retracted towards the guide bearing 226 to enable loading of the vial 100 into the cradle 204.

There now follows a description of an operating sequence, provided by way of example, of the injection kit according to the invention so as to highlight the function and contribution of each individual component.

After insertion of the tubular vial 100 into the cradle 204 according to the modality just described, the operator couples the thrust rod 220 to the piston 1 by screwing it into the threaded seat in the portion 8. In this way, the piston 1 is rendered fixed in translation with respect to the thrust rod 220 in both axial directions, the distal-to-proximal direction and the proximal-to-distal direction.

The operator then couples the proximal unit 210 to the proximal end 206 of the cradle 204: the result of this operation is perforation of the diaphragm 110 by the end 218 of the needle 212 and subsequent divarication by the stud 220. The volume V100 is hence set in fluid communication with the proximal end 216 of the needle 212, and administration of the liquid contained therein can start by moving the slider 228 in the axial direction indicated by the arrow X228 in Figure 6.

Figure 6A illustrates with a dashed and double-dotted line the initial position of the piston 1 and with a solid line an intermediate position of the piston 1 itself, prior to interaction between the axial extension 4 and the distal stud 220 of the jacket 215.

Figure 6 and Figure 6A then represent an incipient stroke of injection of the liquid and correspond in particular to a situation where the end 216 of the needle 212 has penetrated into the relevant anatomical site of the patient (for example, the oral cavity of the patient in the case of dental applications).

The axial extension 4 is configured for co-operating with the stud 220 of the jacket 215 to provide a non-disengageable snap-action coupling with the jacket 215 itself and - as a result - with the needle 212 in order to re-cap the contaminated needle 212 at the end of injection.

In greater detail, the injection kit 200 is sized in such a way that at the end of the injection stroke coupling between the axial extension 4 and the stud 220 takes place. Coupling is provided with the end 216 still in the injection site in order to prevent premature exposure of the contaminated end of the needle.

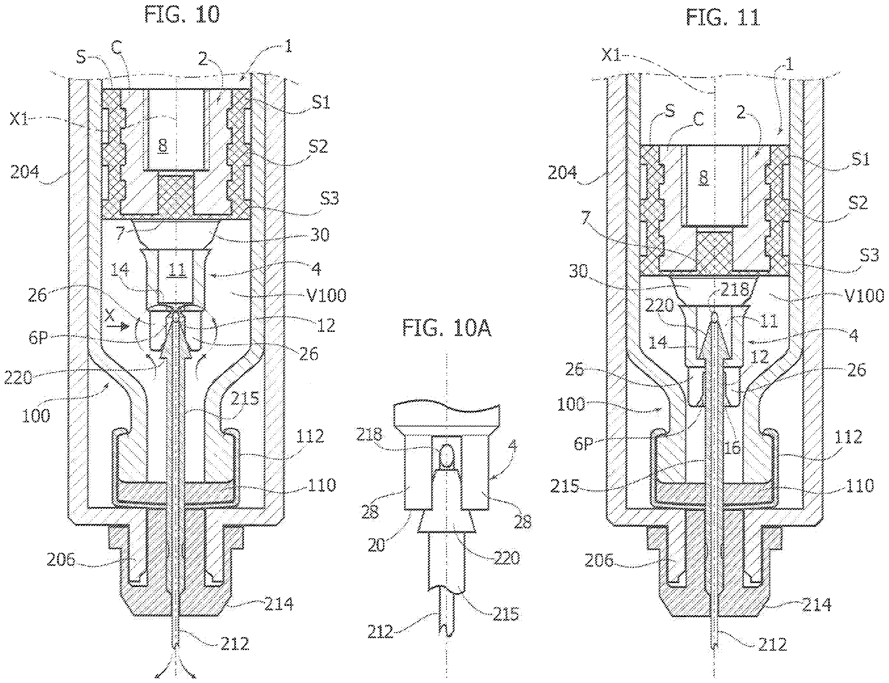

For this purpose, the operator moves the slider 228 in the distal-to-proximal direction, bringing the end 20 of the axial extension 4 towards the stud 220 until the former passes beyond the latter (Figures 7, 7A, 11). In this way, the stretch 12 is traversed by the stud 220, which terminates its own movement within the stretch 11 (and it clicks into position when it passes beyond the stretch 11 with smaller diameter).

Once the stud 220 is in the stretch 11, the coupling between the needle 212 and the piston 1 is irreversible: the stud 220, by axially bearing upon the shoulder 14, renders axial separation of the piston 1 from the jacket 215 and from the needle 212 impossible.

As described at the outset, this is the most critical step of the entire intervention in so far as any liquid that might remain in the volume V100 at the end of the injection stroke hinders any further movement necessary for coupling between the axial extension and the stud 220.

If the piston 1 were without the openings 26, at the moment of the contact between the stud 220 and the end 20, the end 218 of the needle 212 would be isolated from the volume V100 and the liquid would be effectively confined between the surface of influence of the piston 1 (front ring of the skirt 2 and outer surface 4A of the axial extension 4) without any possibility of further advance of the piston 1 on account of the incompressibility of the liquid itself.

The piston 1 according to the invention - and as a result the vial 100 and the kit 200 - are free from the above problem at least for the reasons listed below.
i) The openings 26 create a bypass channel that maintains the fluid communication between the end 18 and the internal volume of the cavity 6. With reference to Figures 10 and 10A, at the moment of contact between the stud 220 and the end 20 of the axial extension 4, the distal end 218 of the needle 212 remains exposed to the portion 6P of the cavity 6 through the one or more openings 26, which are provided exactly in the critical area, where occlusion of the needle and confinement of the residual liquid occurs. It should be noted, in particular in the detailed view of Figure 10A, how the end 218 remains exposed through the openings 26: in this way, the liquid that remains in the volume V100 can evacuate through the needle 212.
ii) The openings 30 constitute a wide bypass channel, which enables evacuation of the entire volume of residual liquid through the needle 212;
iii) The openings 26, unlike the openings 30, do not present any undercut at the moment of removal of the piston 1 from the corresponding mould, whereas the openings 30 certainly create one or more undercuts on account of the closed perimeter and the transverse orientation. Hence, the openings 26 can be provided by means of reliefs directly present on the mould (die or punch), without any need for die slides as in the case of the openings 30. Since die slides present inevitable operating play with respect to the surrounding parts of the mould, the injected plastic material can penetrate into the gaps resulting from the play creating moulding diaphragms or burrs that occlude, either totally or partially, the openings 30, thus neutralising the effectiveness thereof. The openings 26 are, instead, completely immune from this problem.
iv) Even in the worst case in which the openings 30 were to be completely occluded, the opening or openings 26 would be perfectly able to dispose of the volume of residual liquid necessary to complete the stroke of coupling through the needle 212.
v) The piston 1 can operate perfectly even when the openings 30 are intentionally omitted. This simplifies considerably production of the piston 1 and reduces the costs thereof, without significantly reducing the effectiveness thereof.
vi) In combination with what has been stated in points i) to v) above, the openings 26 likewise ensure a certain deformability for the axial end 20 of the piston 1 that is engaged by the stud 220. In particular, they enable a certain divarication of the cylindrical portions 28, reducing the effort necessary for enabling the axial extension 4 to pass beyond the stud 220. This deformability moreover facilitates bleeding of liquid along the needle 212 and around the jacket 215 given that the deformation of the cylindrical portions 28 in an external radial (centrifugal) direction modifies the conditions of interference between the stud 220 and the stretch 12 of the cavity 6P and in particular increases the play existing between them.

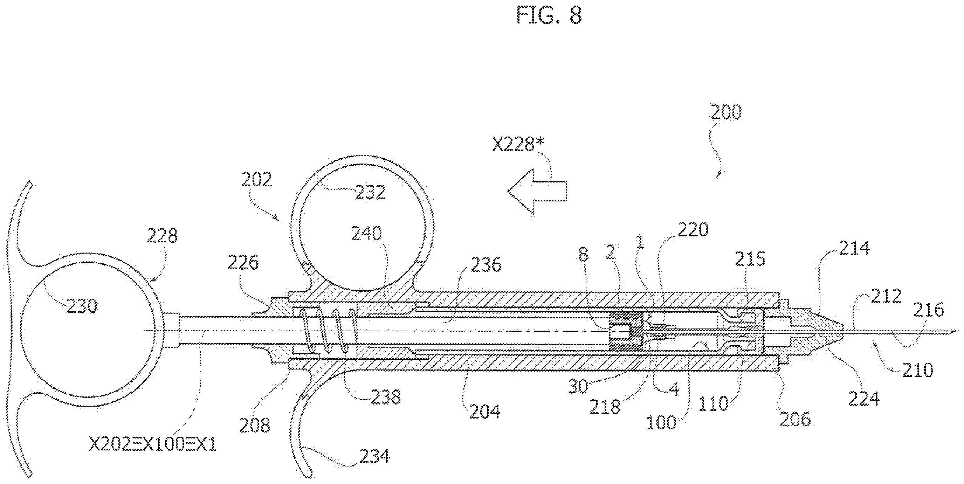

With reference to Figure 8, once the condition of irreversible coupling between the piston 1 and the needle 212 is achieved, the operator can then retract the latter into the volume V100 of the tubular vial 100. To obtain this, the operator retracts (proximal-to-distal movement) the slider 228 in the direction indicated by the arrow X228* in Figure 8. This recalls the piston 1 in a distal direction towards the end 106 and progressively draws the needle 212 into the volume V100. In this regard, the coupling between the bushing member 214 and the jacket 215 is released since the loading threshold is exceeded via movement of the slider.

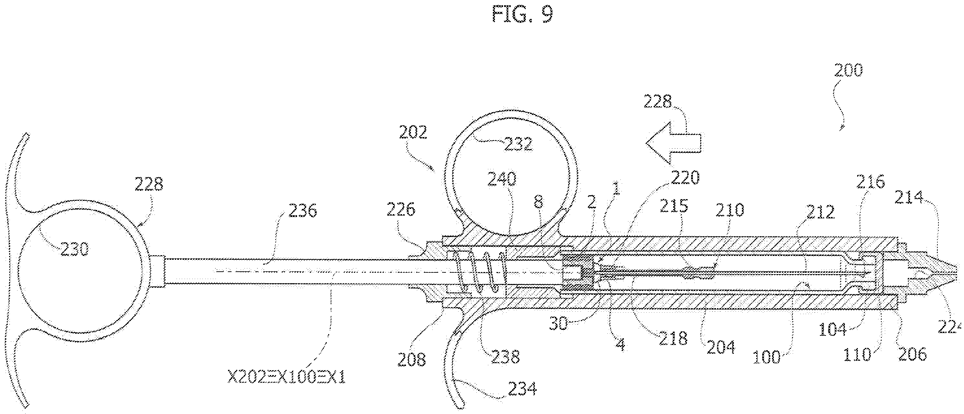

The final condition is illustrated in Figure 9, which represents complete encapsulation of the needle 212 within the volume V100, with the slider 228 in the condition close to that of maximum retraction. The tubular vial 100 can then be extracted from the cradle 204 in total safety, and the bushing member 214, which is by now without the needle 212, can be removed and disposed of. The syringe 202 is ready to be sent on for sterilisation in autoclave with a view to further use thereof.

The person skilled in the branch will hence appreciate that, thanks to the piston 1 according to the invention, it is possible to provide both a tubular vial 100 and an injection kit 200 that are intrinsically safe in the post-injection step. The needle 212 can be encapsulated without the hands of the operator having to interact therewith, but capture and subsequent encapsulation thereof is carried out remotely by acting on the slider 228. Thanks to the piston 1 and to the openings 26, any malfunctioning that would necessitate recourse to direct intervention on the needle 212 is ruled out.

Of course, the details of construction and the embodiments may vary widely with respect to what has been described and illustrated herein, without thereby departing from the scope of the present invention as defined in the annexed claims.

## Claims

1. An injection kit (200) comprising:
- a re-usable syringe (202);
- a tubular vial (100);
- a needle (212) mounted within a through cavity (224) of a bushing member (214) that can be coupled to said re-usable syringe (202);
said tubular vial (100) comprising:
- a tubular casing (102);
- a diaphragm (110) at a first end (104) of said tubular casing (102), and
- a piston (1) sealingly arranged within said tubular casing (102) and axially movable (X100) between a second end (106) of said tubular casing (102), opposite to said first end (104), and said first end (104);
said piston (1) for a tubular vial (100), comprising:
- a longitudinal axis (X1);
- a skirt (2) coaxial to said longitudinal axis (X1) and configured for guiding the piston along an inner surface (102B) of a tubular vial (100); and
- an axial extension (4) comprising an axial cavity (6P) and including an outer surface (4A) and an inner surface (4B), the inner surface (4B) facing said axial cavity (6P), wherein:
said axial extension (4) comprises at least one through opening (26) extending from said outer surface (4A) to said inner surface (4B);
the intersection of said at least one through opening (26) with said outer surface (4A) has an open perimeter; and
the intersection of said at least one through opening (26) with said inner surface (4B) has an open perimeter;
**characterized in that**
said axial extension moreover comprises at least one further through opening (30) extending from said outer surface (4A) to said inner surface (4B), and wherein:
the intersection of said at least one further through opening (30) with said outer surface (4A) has a closed perimeter; and
the intersection of said at least one further through opening (30) with said inner surface (4B) has a closed perimeter;
- said needle is configured for irreversibly coupling with said piston (1) upon a displacement of said piston in a direction proceeding from the second end (106) of said tubular casing (102) towards the first end (104) of said tubular casing (102);
- said needle (212) comprises a proximal end (216) configured for penetration into the body of a patient and a distal end (218) configured for penetration into the diaphragm (110) of the tubular vial (100); and
- said needle (212) further comprises a jacket (215) including a distal stud (220) having a shoulder, said shoulder being configured for bearing upon a shoulder (14) provided in the axial cavity (6P) of said axial extension (4), and wherein said distal end (218) of the needle (212) protrudes over said distal stud (220),
and further wherein a free end (20) of the axial extension (4) of said piston (1) is configured for overstepping said distal stud (220) upon coupling with said needle (212) .

2. The injection kit (200) according to Claim 1, wherein said at least one through opening (26) is provided at a free end (20) of said axial extension (4).

3. The injection kit (200) according to Claim 1 or Claim 2, comprising a pair of through openings (26) at a free end (20) of said axial extension (4), in particular in diametrically opposite positions.

4. The injection kit (200) according to any one of the preceding claims, wherein said axial cavity (6P) comprises a shoulder (14).

5. The injection kit (200) according to any one of the preceding claims, wherein said axial extension (4) comprises a tapered portion (22) adjacent to said skirt (2), and a cylindrical portion (24) adjacent to said tapered portion (22) and extending from said tapered portion (22) to said free end (20).

6. The injection kit (200) according to any one of the preceding claims, wherein each through opening (26) has an axial length (L26) measured along said longitudinal axis (X1) comprised in the range from 2 mm to 3 mm, and a transverse extension (W26) comprised in the range 0.3 mm to 0.7 mm.

## Patentansprüche

1. Ein Injektions-Set (200), welches jeweils Folgendes umfasst:
- eine wiederverwendbare Spritze (202);
- ein röhrenförmiges Fläschchen (100);
- eine Nadel (212), die in einen durchgehenden Hohlraum (224) eines Buchsenelements (214) montiert ist, das mit der genannten wiederverwendbaren Spritze (202) verbunden werden kann;
wobei das genannte röhrenförmige Fläschchen (100) jeweils Folgendes umfasst:
- eine röhrenförmige Hülse (102);
- eine Membran (110) an einem ersten Ende (104) der genannten röhrenförmige Hülse (102) und
- einen Kolben (1), welcher abdichtend in der genannten röhrenförmigen Hülse (102) angeordnet und jeweils axial beweglich (X100) zwischen einem zweiten Ende (106) der genannten röhrenförmigen Hülse (102), und zwar gegenüber dem genannten ersten Ende (104), und dem genannten ersten Ende (104) ist;
wobei der genannte Kolben (1) für ein röhrenförmiges Fläschchen (100) jeweils Folgendes umfasst:
- eine Längsachse (X1);
- einen Schaft (2), welcher koaxial zu der genannten Achse (X1) verläuft und konfiguriert ist, um den Kolben an einer inneren Oberfläche (102B) eines röhrenförmigen Fläschchens (100) entlang zu führen; und
- eine axiale Verlängerung (4), die einen axialen Hohlraum (6P) sowie jeweils eine äußere Oberfläche (4A) und eine innere Oberfläche (4B) umfasst, wobei die innere Oberfläche (4B) dem genannten axialen Hohlraum (6P) gegenüberliegt, wobei:
die genannte axiale Verlängerung (4) mindestens eine durchgehende Öffnung (26) umfasst, welche sich jeweils von der genannten äußeren Oberfläche (4A) zu der genannten inneren Oberfläche (4B) erstreckt;
- die Überschneidung der genannten mindestens einen durchgehenden Öffnung (26) mit der genannten äußeren Oberfläche (4A) einen offenen Umriss aufweist; und
- die Überschneidung der genannten mindestens einen durchgehenden Öffnung (26) mit der genannten inneren Oberfläche (4B) einen offenen Umriss aufweist;
**dadurch gekennzeichnet, dass**
die genannte axiale Verlängerung darüber hinaus mindestens eine weitere durchgehende Öffnung (30) aufweist, welche sich jeweils von der genannten äußeren Oberfläche (4A) zu der genannten inneren Oberfläche (4B) erstreckt, und wobei:
die Überschneidung der genannten mindestens einen weiteren durchgehenden Öffnung (30) mit der genannten äußeren Oberfläche (4A) einen geschlossenen Umriss aufweist; und
die Überschneidung der genannten mindestens einen weiteren durchgehenden Öffnung (30) mit der genannten inneren Oberfläche (4B) einen geschlossenen Umriss aufweist;
- die genannte Nadel konfiguriert ist, um sich bleibend mit dem genannten Kolben (1) zu verbinden, und zwar bei der Verschiebung des genannten Kolbens in einer Richtung, welche jeweils vom zweiten Ende (106) der genannten röhrenförmigen Hülse (102) in Richtung des ersten Endes (104) der genannten röhrenförmigen Hülse (102) verläuft;
- die genannte Nadel (212) jeweils ein proximales Ende (216) umfasst, das für die Penetration in den Körper eines Patienten konfiguriert ist, sowie ein distales Ende (218), das für die Penetration in die Membran (110) des röhrenförmiges Fläschchens (100) konfiguriert ist; und
- die genannte Nadel (212) desweiteren eine entsprechende Schutzhülle (215) umfasst, welche einen distalen Stift (220) mit einer Schulter einschließt, wobei die genannte Schulter ausgestaltet ist um auf einer Schulter (14) getragen zu werden, die in dem axialen Hohlraum (6P) der genannten axialen Verlängerung (4) vorgesehen ist, wobei das genannte distale Ende (218) der Nadel (212) über den genannten distalen Stift (220) hinaus vorsteht,
- und wobei desweiteren ein freies Ende (20) der axialen Verlängerung (4) des genannten Kolbens (1) konfiguriert ist, um den genannten distalen Stift (220) bei Verbindung mit der genannten Nadel (212) zu überschreiten.

2. Das Injektions-Set (200) gemäß Anspruch 1, wobei die genannte mindestens eine durchgehende Öffnung (26) an einem freien Ende (20) der genannten axialen Verlängerung (4) vorgesehen ist.

3. Das Injektions-Set (200) gemäß Anspruch 1 oder Anspruch 2, welches ein Paar durchgehender Öffnungen (26) an einem freien Ende (20) der genannten axialen Verlängerung (4) umfasst, und zwar insbesondere in diametral entgegengesetzten Stellungen.

4. Das Injektions-Set (200) gemäß einem beliebigen der vorausgegangenen Ansprüche, wobei der genannte axiale Hohlraum (6P) eine entsprechende Schulter (14) umfasst.

5. Das Injektions-Set (200) gemäß einem beliebigen der vorausgegangenen Ansprüche, wobei die genannte axiale Verlängerung (4) jeweils einen keilförmigen Abschnitt (22) umfasst, welcher an dem genannten Schaft (2) anliegt, sowie einen zylindrischen Abschnitt (24), der an dem genannten keilförmigen Abschnitt (22) anliegt und sich jeweils von dem genannten keilförmigen Abschnitt (22) zu dem genannten freien Ende (20) erstreckt.

6. Das Injektions-Set (200) gemäß einem beliebigen der vorausgegangenen Ansprüche, wobei jede durchgehende Öffnung (26) jeweils eine axiale, an der genannten Längsachse (X1) gemessene Länge (L26) aufweist, welche in einem Bereich zwischen 2 mm bis 3 mm liegt, sowie eine Querausdehnung (W26), die in einem Bereich zwischen 0,3 mm bis 0,7 mm liegt.

## Revendications

1. Kit d'injection (200) comprenant:
- une seringue réutilisable (202);
- un flacon tubulaire (100);
- une aiguille (212) montée dans une cavité traversante (224) d'un élément de douille (214) qui peut être couplé à ladite seringue réutilisable (202) ;
ledit flacon tubulaire (100) comprenant:
- une enveloppe tubulaire (102);
- un diaphragme (110) à une première extrémité (104) de ladite enveloppe tubulaire (102), et
- un piston (1) disposé de manière étanche à l'intérieur de ladite enveloppe tubulaire (102) et axialement mobile (X100) entre une seconde extrémité (106) de ladite enveloppe tubulaire (102), opposée à ladite première extrémité (104), et ladite première extrémité (104);
ledit piston (1) pour un flacon tubulaire (100), comprenant:
- un axe longitudinal (X1);
- une jupe (2) coaxiale audit axe longitudinal (X1) et configurée pour guider le piston le long d'une surface intérieure (102B) d'un flacon tubulaire (100); et
- une extension axiale (4) comprenant une cavité axiale (6P) et incluant une surface extérieure (4A) et une surface intérieure (4B), la surface intérieure (4B) faisant face à ladite cavité axiale (6P),
dans lequel:
ladite extension axiale (4) comprend au moins une ouverture traversante (26) s'étendant de ladite surface extérieure (4A) à ladite surface intérieure (4B) ;
l'intersection de ladite au moins une ouverture traversante (26) avec ladite surface extérieure (4A) a un périmètre ouvert; et
l'intersection de ladite au moins une ouverture traversante (26) avec ladite surface intérieure (4B) a un périmètre ouvert;
**caractérisé en ce que**
ladite extension axiale comprend en outre au moins une autre ouverture traversante (30) s'étendant de ladite surface extérieure (4A) à ladite surface intérieure (4B), et dans lequel: l'intersection de ladite au moins une autre ouverture traversante (30) avec ladite surface extérieure (4A) a un périmètre fermé; et
l'intersection de ladite au moins une autre ouverture traversante (30) avec ladite surface intérieure (4B) a un périmètre fermé;
- ladite aiguille est configurée pour un couplage irréversible avec ledit piston (1) lors d'un déplacement dudit piston dans une direction allant de la seconde extrémité (106) de ladite enveloppe tubulaire (102) vers la première extrémité (104) de ladite enveloppe tubulaire (102);
- ladite aiguille (212) comprend une extrémité proximale (216) configurée pour pénétrer dans le corps d'un patient et une extrémité distale (218) configurée pour pénétrer dans le diaphragme (110) du flacon tubulaire (100); et
- ladite aiguille (212) comprend en outre un étui (215) incluant une pointe distale (220) ayant un épaulement, ledit épaulement étant configuré pour prendre appui sur un épaulement (14) prévu dans la cavité axiale (6P) de ladite extension axiale (4), et dans laquelle ladite extrémité distale (218) de l'aiguille (212) fait saillie sur ladite pointe distale (220),
et en outre dans lequel une extrémité libre (20) de l'extension axiale (4) dudit piston (1) est configurée pour dépasser ladite pointe distale (220) lors du couplage avec ladite aiguille (212).

2. Kit d'injection (200) selon la revendication 1, dans lequel ladite au moins une ouverture traversante (26) est prévue à une extrémité libre (20) de ladite extension axiale (4).

3. Kit d'injection (200) selon la revendication 1 ou la revendication 2, comprenant une paire d'ouvertures traversantes (26) à une extrémité libre (20) de ladite extension axiale (4), en particulier dans des positions diamétralement opposées.

4. Kit d'injection (200) selon l'une quelconque des revendications précédentes, dans lequel ladite cavité axiale (6P) comprend un épaulement (14).

5. Kit d'injection (200) selon l'une quelconque des revendications précédentes, dans lequel ladite extension axiale (4) comprend une partie conique (22) adjacente à ladite jupe (2), et une partie cylindrique (24) adjacente à ladite partie conique (22) et s'étendant de ladite partie conique (22) à ladite extrémité libre (20).

6. Kit d'injection (200) selon l'une quelconque des revendications précédentes, dans lequel chaque ouverture traversante (26) a une longueur axiale (L26) mesurée le long dudit axe longitudinal (X1) comprise dans la gamme de 2 mm à 3 mm, et une extension transversale (W26) comprise dans la gamme de 0,3 mm à 0,7 mm.
